# EUROPEAN PATENT APPLICATION

(11) **EP 2 561 845 A1**
(43) Date of publication of application: **27.02.2013**
(21) Application number: 11178298.3
(22) Date of filing: 22.08.2011
(51) Int. Cl.: A61F 13/15, A61F 13/56, A61F 13/62

(54) **Disposable diaper ear in the form of a continuos strip having a fastener tab exhibiting no fiber contamination on a spool**

(71) Applicant: BENTO bantcilik ve Temizlik Maddeleri Sanayi Ticaret A.S., 34909 Istanbul (TR)
(72) Inventor: Kus, Ercan, 34909 Istanbul (TR)
(74) Representative: Sevinç, Erkan

(57) **Abstract**

The present invention proposes a closure system for effecting closure of a diaper around the abdomen of a wearer, said closure system being provided as a continuous strip in the form of a central region, a first lateral region and a second lateral region. Each of said central and lateral regions, extend along the length of said continuous strip in the machine direction. Said first lateral region is disposed on one longitudinal side of said central region and said second lateral region is disposed on the other longitudinal side of the central region. Each lateral region comprises a laminate of elastic and non-elastic layers wherein said central portion comprises a non-woven layer joined to a mechanical fastener portion. Said mechanical fastener portion contacts a non-fibrous upper surface of a lower layer non-woven layer on a spool of said continuous strip.

## Description

### Technical Field of the Invention

The invention relates to an all-in-one manufactured easily attachable closure tape for diapers combined with elastic ear. The closure tape of the present invention comprises a mechanical fastener portion for fastening the ear to a landing zone location of a diaper.

### Background of the Invention

There have been a wide variety of products in the market for fastening incontinence garments such as diapers or nappies for adults and babies since at least half a century. These have been generally in the form of pressure sensitive adhesive tapes and more recently mechanical fastening systems derived from the Velcro® products comprising a hook and a loop element.

Some closure systems relying solely on the pressure sensitive adhesive coating of a thermoplastic tape have also been introduced in the market. Such tapes transfer one layer of their thermoplastic components onto the surface of a diaper and rely merely on the pressure sensitive adhesive of the transferred thermoplastic layer to hold the diaper properly in place. The transferred adhesive coated thermoplastic layer generally has a relatively small area and provides extremely poor results especially on baby and/or adult diapers, where maintaining tightness requires more shear strength of fastening material. These closure systems are very much restricted on the number of times the diaper is re-opened and closed due to the degradation of the tack of the adhesive, even if the user pays extreme attention to the cleanliness of the conditions. The degradation in the tack of the adhesive becomes obsolete, if the closure tape is handled with dirty or greasy fingers of the user.

Mechanical fastening systems rely on a hook affixed to a closure tape, in turn affixed onto the ear or side of a diaper. The loop material is usually in the form of a frontal tape which is also designated as a landing zone. When hook material is pressed against the loop material, the resulting bond is satisfactory in terms of its shear and peel strength and closure of the diaper around the abdomen of the wearer can be securely achieved.

A mechanical fastener system may comprise a hook strip affixed to the underside of a fastening tab. When the fastening tab is lifted from the side or ear of a diaper in order to close the diaper around a wearer's abdomen, its hook grips onto the nonwoven (or the like) substrate placed on the front surface of the diaper.

Ears are mostly fully or partially elasticized side panels that are attached to the upper left and right regions of diapers, with the purpose of making diaper fit snuggly and comfortably around the baby/adult torso, and come in various shapes and dimensions, for example and not limited to, trapezoidal, squares, oblong or other similar geometrical shapes or more elaborate versions of these. Area of each such ears varies from 10 to 60 cm²_{,} or for adult diapers from 10 to 100 cm².

Diaper ears currently available on the market can be mounted on the left/right sides of the diaper by means of adhesives or ultrasonic welding, or both. In addition to the ears so mounted on diapers, the ears themselves must be coupled with side tabs (or fastening tabs) containing hooks. Consequently the mounting of these items on the diaper is achieved by means of complicated and expensive pieces of machinery that cut the ears to specific shapes and then couples it to the side tapes and then transports these to be ultimately positioned and stuck or welded onto the diapers' left and right sides. Machinery that can do all that does increase the basic cost of a diaper line by anything from 25-50%.

The ears according to the present invention feature a middle section with specially coated or non-coated smooth surfaced non-woven that does not infringe on the efficiency of the hook portion. The hooks of the invention were therefore not prone to "fiber contamination" when supplied in rolls. In other words the hook portion of the spool would not stick to the fibers of the substrate that carried the hooks.

The mechanical fastener system according to the present invention do not require extensive machinery as they come all assembled in the manner to feature a mechanical fastening tab adapted to be joined to an ear portion as a substrate in the form of a laminate. Said mechanical fastening tab typically engages with a second mechanical fastener component so as to effect assembling of the product on the wearer. Said ear laminate comprises a film material being attached to a non-woven material. The hook material generally comprises a base sheet material from which stem-like projections extend.

The spooling as an essential ingredient of the present invention, not only is an economical way of handling/transporting the product, but is also of crucial importance. Spooling machines are readily available worldwide from various manufacturers. Spooling machines are widely used on textile machines for the purpose of carrying long lengths of threads in a compact packaging and for easy unwinding. Spooling machines spoolers move laterally - on a horizontal plane- against the incoming thread/strip in coordination with the speed and the desired angle of spooling. The feeding of threads/strips onto the spools is often helped by an arm moving across the surface of the spooler, which works in tandem with the spooling part of the machine.

### Objects of the Invention

An object of the present invention is to provide a multi layered closure tape that eliminates drawbacks of fastening systems known in the prior art.

Another object of the present invention is to provide a reliable closure system for use with adult or baby diapers, which closure system comprises a mechanical fastening strip that enables the user to repeatedly and effectively re-open and close and even adjust the incontinence garment to which the closure system is attached.

Another object of the present invention is to provide a diaper ear with a fastener tab requiring no complicated or expensive pieces of machinery for effecting mounting of said diaper ear.

Another object of the present invention is to provide a diaper ear with a fastener tab having a hook portion not subject to fiber contamination when supplied in spools.

Another object of the present invention is to provide a method for manufacturing diaper ears in the form of a continuous strip for making a plurality of the side panels.

Another object of the invention is to supply the diaper ear continuous strip of the invention in the form of spools, onto which said strip is wound either as a combined left and right continuous strip or alternatively pre-cut in 2 halves and each half - left and right- being wound on separate spools each containing half of the continuous strip.

### Summary of the Invention

The present invention proposes a closure system for effecting closure of a diaper around the abdomen of a wearer, said closure system being provided as a continuous strip in the form of a central region, a first lateral region and a second lateral region.

Each of said central and lateral regions, extend along the length of said continuous strip in the machine direction. Said first lateral region is disposed on one longitudinal side of said central region and said second lateral region is disposed on the other longitudinal side of the central region. Each lateral region comprises a laminate of elastic and non-elastic layers wherein said central portion comprises a non-woven layer joined to a mechanical fastener portion. Said mechanical fastener portion contacts a non-fibrous smooth upper surface of a lower layer non-woven layer on a spool of said continuous strip.

### Brief Description of Figures

Fig. 1 demonstrates a schematic view of a closure means for a diaper according to the present invention in the form of layers.
Fig. 2 demonstrates the upper schematic view of the continuous strip comprising the ears with zero material waste in manufacturing stage according to the present invention.
Fig. 3 demonstrates the upper schematic view of the continuous strip comprising the ears with zero material waste in manufacturing stage according to another embodiment of the present invention.
Fig. 4 demonstrates the upper schematic view of the continuous strip comprising the ears with optimized material waste in manufacturing stage according to another embodiment of the present invention.

### Detailed Description of the Invention

The present invention provides a closure system with a fastening tab effectively incorporating a hook portion into an ear section that comes into contact with a frontal tape (or loop portion) in order to close the diaper around the wearer body.

The multi-layered closure system according to the present invention is produced, stored and supplied on a spool which carries a certain length of tape and is designed for continuous feed into a diaper manufacturing line. The required length of tape is cut at right angles to the spool by the diaper manufacturing line and is affixed onto the ears or sides of the diaper. Once the multi-layered closure system of the present invention is affixed to its locations on the ears of a diaper, the end user typically lifts the top layers of the closure system. The user affixes the top layer onto a desired location on the front of the diaper pressing firmly on it.

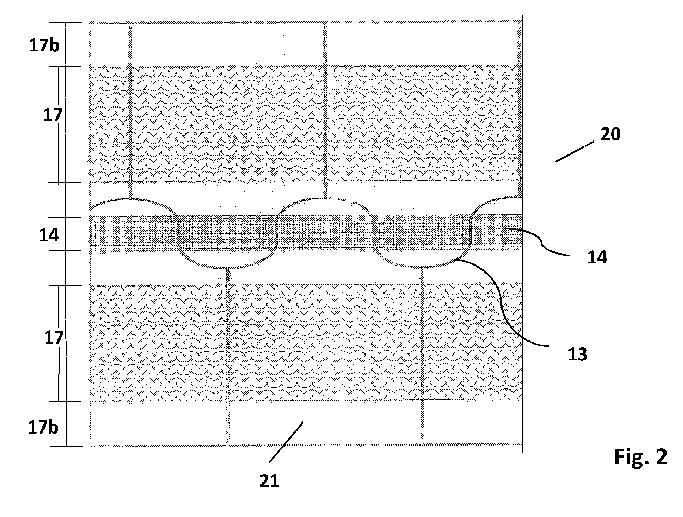

The closure system in the form of a diaper ear with a fastening tab according to the present invention provides a construction of a continuous strip generally designated 20 as shown in Fig. 2, which is adapted to be joined to a diaper chassis after being cut in the form of individual ear cells (21) having an elastic portion (17) and a mechanical fastener portion (14). Each individual ear cell (21) is separated from a neighboring cell (21) by a cell border (13) to be used as a cutting line for later cutting of ear cells (21) to be joined to diapers.

The continuous strip (20) comprises a central region (25), a first lateral region (23a), and a second lateral region (23b). Said central and lateral regions (23a, 25, 23b), each run the length of said continuous strip (20) in the machine direction, with the first lateral region 23a being located on one longitudinal side of said central region (25) and the second lateral region (23b) being located on the other longitudinal side of the central region (25).

The distal longitudinal edge of the first lateral region (23a) and the distal longitudinal edge of the second lateral region (23b) define the width of the continuous strip (20) in the cross direction. The first lateral region (23a) and the second lateral region (23b) can have substantially the same width in the cross direction and/or mirror each other relative to the central region (25). In this manner, a first ear cell (21) to be joined to a diaper chassis will have the same construction as a second ear cell neighboring the same.

According to the present invention, said lateral regions (23a, 23b) comprise a non-woven fabric (11) and an elastic film (12) laminate. With particular reference to an elastic film laminate, it can comprise, for example, an elastic film layer (12) with a non-woven fabric layer (11) on one or both sides.

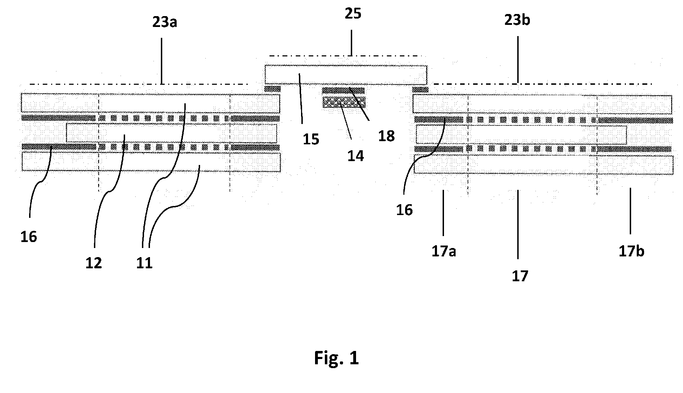

The elastic region (17) according to the present invention can comprise a laminate of elastic (12) and non-elastic layers (11) as demonstrated in Fig. 1, with said non-elastic layers (11) being activated to render them elastic. Fig. 1 demonstrates an elastic region (17) comprising an elastic layer (12) sandwiched between two non-elastic layers (11) such as non-elastic non-woven layers (11) by means of adhesive layers (16), with the non-elastic layers (11) being activated in a conventional manner, as is known to the skilled person in the art. Said activated region (17) is neighboring two regions (17a, 17b) at each lateral regions (23a, 23b), inner neighboring regions (17a) extending at both sides of said hook portion (14).

It is known that the hooks (14) being contaminated to any degree considerably lose their efficiency in respect of a loop region. As the closure system in the form of a continuous strip (20) according to the invention is designed for continuous feed into a diaper manufacturing line, it is an essential aspect in this manner that the ears (21) containing hooks (14) on fastening tabs (25) that are directly stuck onto the ear portions (21) do not come into contact with their underside when rolled. This ensures that the hooks (14) are not prone to fiber contamination.

Considering that the ears (21) are made by sandwiching one layer of elastomeric (12) between the upper and lower layers (11) of non-woven, and such non-woven layers (11) being fibrous as they need to stick to the elastomeric (12) and to each other, the central portion (25) being basically a smooth-surfaced non-woven and having no fibrous finish on its surface according to the present invention ensures that no contamination occurs.

The mechanical fastener portion (14) is adhered to a smooth-surfaced non-woven layer (15) and is designed in the form of a hook or microplast® strip preferably around 8-14 mm wide; being affixed to the underside of said fastening tab (25). This is typically achieved by means of an adhesive layer (18) applied on the underside of said tab (25). Various types of hook material can be used to form the hook tabs of the present closure tape. They are generally made of an extruded sheet or strip containing large numbers (around 250 micro hooks per cm²) of mushroom/hexagonal shaped micro hooks on its upper face and is plain on its lower surface. The micro hooks have extensive gripping properties and grip most surfaces to a certain degree, except thermoplastic derivatives such as polyethylene and polypropylene or coextruded films. Optimum gripping properties are achieved when the hook is pressed against a loop material that provide in optimum sheer and peel values. These typically range at least 30 N for sheer values (astmd 5170-91) and at least 1.5 N for peel values (astmd 5170-91) when tested on zwick/ roell z 2.5 tester.

The elastic film (11) according to the present invention may consist of a thermoplastic film of a sole thermoplastic material, conventionally polypropylene or polyethylene or derivatives thereof. The adhesive layers (16, 18) may conventionally be made of generally synthetic polymers or rubber mixed with synthetic resins. The central region (25) is ideally made in non-woven fabric. However, the non-woven fabric can be replaced by any polyolephine film, such as but not limited to; polypropylene film or polyethylene film or mixtures of both.

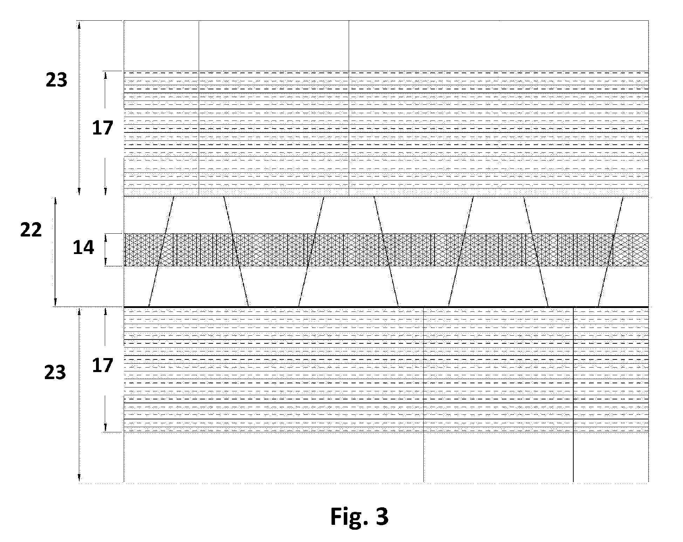

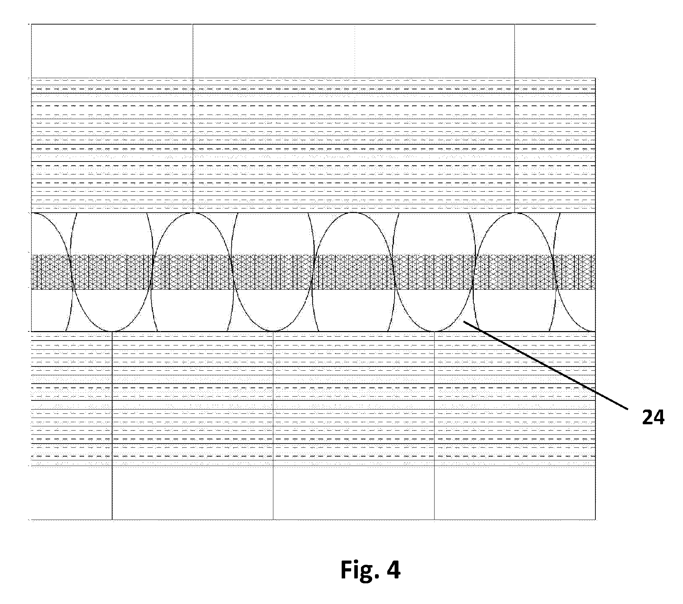

The numeral "22" in Fig. 3 indicates the non-woven portion of the continuous strip (20) which namely consists of the non-woven central region (25) and the two non-woven inner neighboring regions (17a). Further, the numeral "23" indicates carded non-woven portions incorporating said activated region (17) and its outer neighboring regions (17b). On the other hand, Fig. 4 demonstrates the continuous strip comprising the ears with optimized material waste regions (24).

In a nutshell, the present invention proposes a closure system for effecting closure of a diaper around the abdomen of a wearer, said closure system being provided as a continuous strip (20) in the form of a central region (25), a first lateral region (23a) and a second lateral region (23b). Each of said central and lateral regions (23a, 25 and 23b), extend along the length of said continuous strip (20) in the machine direction. Said first lateral region (23a) is disposed on one longitudinal side of said central region (25) and said second lateral region (23b) is disposed on the other longitudinal side of the central region (25). Each lateral region (23a, 23b) comprises a laminate of elastic (12) and non-elastic layers (11) wherein said central portion (25) comprises a non-woven layer (15) joined to a mechanical fastener portion (14). Said mechanical fastener portion (14) contacts a smooth-surfaced non-fibrous upper surface of a lower layer non-woven layer (15) on a spool of said continuous strip (20).

## Claims

1. A closure system for effecting closure of a diaper around the abdomen of a wearer, said closure system being provided as a continuous strip (20) in the form of a central region (25), a first lateral region (23a) and a second lateral region (23b), said central and lateral regions (23a, 25 and 23b), each extending along the length of said continuous strip (20) in the machine direction, with the first lateral region (23a) being disposed on one longitudinal side of said central region (25) and the second lateral region (23b) being disposed on the other longitudinal side of the central region (25), each lateral region (23a, 23b) comprising a laminate of elastic (12) and non-elastic layers (11) **characterized in that** said central portion (25) comprises a non-woven layer (15) joined to a mechanical fastener portion (14) such that said mechanical fastener portion (14) contacts a smooth-surfaced non-fibrous upper surface of a lower layer non-woven layer (15) on a spool of said continuous strip (20).

2. A closure system for effecting closure of a diaper around the abdomen of a wearer as set forth in Claim 1 wherein said first and second lateral regions (23a, 23b) are made by sandwiching one layer of elastomeric (12) between an upper and a lower layer of fibrous non-elastic non-woven layers (11).

3. A closure system for effecting closure of a diaper around the abdomen of a wearer as set forth in Claim 1 or 2 wherein said continuous strip (20) comprises ear cells (21) adapted to be cut by a cell border (13) to be joined to a diaper chassis, in the form of individual ear cells (21) substantially having the same construction, said ear cells (21) being adapted to be cut in the form of individual ear cells (21) having an elastic portion (17) and a mechanical fastener portion (14).

4. A closure system for effecting closure of a diaper around the abdomen of a wearer as set forth in Claim 2 wherein said elastic non-elastic non-woven layers (11) are activated.

5. A closure system for effecting closure of a diaper around the abdomen of a wearer as set forth in Claim 4 wherein said non-woven central region (25) and said two non-woven inner neighboring regions (17a) forms a central strip (22) adapted to be cut such that each cell (21) has the same construction in terms of geometrical layout of different material character regions.

6. A closure system for effecting closure of a diaper around the abdomen of a wearer as set forth in any previous Claims wherein said non-woven layers (11, 15) are silicone coated and UV cured.
